# NEUE EUROPÄISCHE PATENTSCHRIFT

(11) **EP 0 652 017 B2**
(45) Veröffentlichungstag und Bekanntmachung des Hinweises auf die Entscheidung über den Einspruch: **07.01.2004**
(45) Hinweis auf die Patenterteilung: 08.09.1999
(21) Anmeldenummer: 94115691.1
(22) Anmeldetag: 05.10.1994
(51) Int. Cl.: A61L 33/00

(54) **Beschichtung für Biomaterial**
Coating for biomaterial
Revêtement pour biomatériau

(30) Priorität: 07.10.1993 DE 4334272
(43) Veröffentlichungstag der Anmeldung: 10.05.1995
(73) Patentinhaber: Stemberger, Axel, Dr., D-85579 Neubiberg (DE)
(72) Erfinder: Stemberger, Axel, Dr., D-85579 Neubiberg (DE)
(74) Vertreter: Haft, von Puttkamer, Berngruber, Czybulka

(56) Entgegenhaltungen:
- EP-A- 0 562 612
- EP-A- 0 578 998
- EP-B- 0 237 037
- WO-A-89/01791
- WO-A-93/06792
- DE-C- 3 913 926
- GB-A- 2 166 977
- US-A- 3 663 288

## Beschreibung

Die Erfindung bezieht sich auf eine Beschichtung für sogenanntes Biomaterial. Als Biomaterial wird ein Material bezeichnet, das in den Blutstrom oder das Gewerbe des menschlichen Körpers eingebracht wird, so z.B. Infusionskatheter, Herzkatheter, Ballonkatheter, Elektroden, Nahtmaterial für Gefäßanastomosen, Gefäßprothesen oder Stützkörper für Gefäße, sogenannte Stents, etc., die kurzzeitig wie auch längerfristig unmittelbar in Arterien und Venen sowie in Körpergewebe verbracht werden. So dienen z.B. die mittels komplizierter Kathetersysteme unter Röntgenkontrolle meistens in die Coronararterien eingebrachten Stents nach Aufweitung des verengten Bereiches der Arterie zur inneren Schienung des Gefäßes und verbleiben zu diesem Zwecke lebenslang an ihrer Stelle. Die Gefährdungen der Patienten, z.B. durch Thrombosenbildung und Entzündungen, sind bekannt und hinsichtlich Therapieerfolg mit dem Schweregrad der Erkrankung abzuwägen.

Nach dem Einbringen von Biomaterial aus Metall und/oder verschiedenen Kunststoffen in den Blutstrom, so z.B. beim Einführen eines Katheters, kann es zu einer lokal begrenzten und teilweise generalisierten Aktivierung der Blutgerinnung an dem Biomaterial kommen. Bekannt ist, daß gebundene Proteine des Blutplasmas, sogenannte adhäsive Proteine, wie Albumin, und Fibrinbeläge, die Adhäsion und Aggregation von Thrombozyten an dem Biomaterial unterhalten. Die für die gegenseitige Haftung der Proteine verantwortlichen Peptidstrukturen, die sogenannten RGD-Peptide (Glycin, Arginin, Asparagin), sind weitgehend aufgeklärt. Diese Strukturen sind auch an der Haftung der Thrombozyten über entsprechende Rezeptoren der Thrombozytenoberfläche beteiligt.

Des weiteren können Biomaterialien die schützenden Zellschichten, mit denen die Gefäße ausgekleidet sind, verletzen, und die darunter liegenden Bindegewebsstrukturen, wie Kollagen, freilegen. Auch dort erfolgt dann Adhäsion sowie Aggregation der Thrombozyten. Gleichzeitig freigesetztes Thromboplastin aktiviert die plasmatische Gerinnung.

Die Ablagerung von Blutgerinnseln auf Fremdoberflächen ist somit die Folge einer komplexen Reaktionskette plasmatischer und zellulärer Mechanismen der Blutgerinnung, verstärkt durch Wechselwirkungen dieser Systeme. Wenn es durch eine begleitende medikamentöse Behandlung nicht gelingt, die Blutgerinnung ganz oder teilweise aufzuheben bzw. bereits gebildete Gerinnsel aufzulösen, kann dies zu einem lebensbedrohenden Gefäßverschluß führen.

Weiterhin ist bekannt, daß Blutgerinnsel die Wundheilung durch einwandernde polyploide Stammzellen triggern. Als Folge des überschießenden Wachstums sogenannter "glatter Muskelzellen" (smooth muscle cells) kann dann durch dadurch verursachte Stenosen der Blutstrom in den Gefäßen erneut eingeengt werden.

Mit der Implantation von oben erwähnten Stützkörpern für Gefäße, den sogenannten Stents, werden aufgrund der geschilderten Probleme der Blutgerinnung und Entzündung bei vielen Patienten nur kurzfristige Verbesserungen erzielt. Durch Mikrothromben an der Grenzfläche zwischen Gefäßwand und Stent kann es zu einer unerwünschten, überschießenden Gefäßproliferation mit erneuter Verengung des Gefäßes kommen. Eine gleichzeitige systemische Behandlung dieser Patienten mit hohen Dosen von blutgerinnungshemmenden Arzneistoffen, sogenannten Antikoagulantien, wie Heparin oder Cumarinderivaten ist unbedingt notwendig, teilweise in Kombination mit Medikamenten, sogenannten Thrombozytenaggregationshemmern, die eine Hemmung der Thrombozyten bewirken. Auch durch die systemische Gabe neu entwickelter gerinnungshemmender Arzneistoffe, wie Hirudin, entsprechender Analoga sowie von neuentwickelten Hemmstoffen der Thrombozytenaggregation haben sich die Probleme, die mit der Ablagerung von Blutgerinnseln auf den Oberflächen von Biomaterialien verbunden sind, nur marginal verbessert. Die systemische Gabe hoher Dosen von blutgerinnungshemmenden Medikamenten ist mit dem Risiko lebensbedrohender Blutungen innerer Organe und des Gehirns verbunden. Trotz der Antikoagulierung gehören nach derartigen Eingriffen akute Verschlüsse bei 4 bis 10 % der Eingriffe, Restenosen bei 35 % und bedrohliche Blutungen bei 5 bis 10 % zum klinischen Alltag.

Um die Anlagerung von Thromben auf Biomaterialien, so z.B. Katheteroberflächen, zu verhindern, ist es aus der US-PS 4 281 668 bekannt, Biomaterial, in diesem Falle eine implantierbare Kohlenstoffelektrode, mit einer dünnen Beschichtung eines hydrophilen ionenleitenden Kunststoffes zu versehen, wobei dieser Kunststoff selbstverständlich körper-, gewebeund/oder blutverträglich ist.

Eine weitere Problematik ist die Keimbesiedelung von Biomaterial. Adsorptiv gebundene Proteine des Blutplasmas, wie Albumin, generell die sogenannten adhäsiven Proteine, und Blutgerinnsel provozieren die Einnistung von Keimen und weiterhin die Bildung geladener Schleimsubstanzen. Durch diese schleimartigen Überzüge entziehen sich die Bakterien der körpereigenen Abwehr und neutralisieren weiter systemisch verabreichte Antibiotika: Die Empfindlichkeit gegenüber den Bakterien ist somit drastisch herabgesetzt. Mittels intravenöser Antibiotikatherapie können die hohen bakteriziden Konzentrationen nicht immer erreicht werden, da eine Dosiserhöhung häufig aufgrund der Nebenwirkungen kontraindiziert ist.

Nur bei Implantaten aus Biomaterial, die kurzfristig im Körper verweilen, ist durch eine rasche Entfernung des Materials z.B. die Problematik einer Thrombophlebitis beseitigt. Dies gilt in der Regel nicht für mittelfristig bzw. langfristig implantierte Biomaterialien. Jede Reimplantation ist jedoch mit Schmerz sowie erneuter Gesundheitsgefährdung belastet.

Aus der US-PS 5 103 837 ist es bekannt, eine implantierte poröse Stimulationselektrode für einen Herzschrittmacher mit einer dünnen Beschichtung eines hydrophilen Polymers zu versehen, in die ein entzündungshemmendes Steroid eingebettet ist.

Die erwähnten und weitere Lösungsansätze für die Problematik der plasmatischen und zellulären Blutgerinnung sowie der Entzündungsgefährdung betreffen im wesentlichen die Änderung der Oberflächenladung, die Einarbeitung von blutgerinnungshemmenden Arzneistoffen in nicht resorbierbare Kunststoffe oder die Fixierung von Arzneistoffen durch chemische Verfahren. Die Einarbeitung und Fixierung entsprechender Arzneistoffe in eine Kunststoffmatrix brachte jedoch noch nicht die gewünschten Erfolge, da aus den unmittelbaren oberflächennahen Schichten die Arzneistoffe nur kurzfristig und in zu geringen Konzentrationen freigesetzt werden. Diese Oberflächen werden in der Folge im Blutstrom ebenfalls rasch mit Proteinablagerungen sowie Blutgerinnseln überzogen. Durch die Proteinadsorption wird die Freigabe der eingearbeiteten oder auch die Wirkung der chemisch gebundenen Arzneistoffe eingeschränkt oder gar verhindert. Dies führt zu einer erneuten Bildung thrombogener Oberflächen, verbunden mit der Gefahr der Ausbildung von Blutgerinnseln, einer überschießenden Gewebeneubildung und Bakterienbesiedelung.

Aus der DE-PS 3913 926 ist ein Verfahren zur Herstellung einer textilen Gefäßprothese bekannt. Wenn derartige textile Gefäßprothesen im menschlichen Körper eingebaut werden, müssen sie abgedichtet werden, da ansonsten durch die Maschen der Prothese das unter Druck stehende Blut austritt und zu erheblichen Blutverlusten führen kann. Diese Abdichtung erfolgt gemäß der erwähnten Patentschrift durch eine Beschichtung mit einem resorbierbaren Kunststoff, durch den die gesamte textile Prothese sozusagen mit einer zweiten Haut an ihrem Umfang vollständig umgeben wird. In dieser Patentschrift ist allerdings kein Hinweis auf die Verhinderung von Thrombosen enthalten.

Aus der WO 84/00302 ist ein biokompatibles antithrombogenes Material bekannt, welches z.B. zur Herstellung von künstlicher Haut bei der rekonstruktiven Chirurgie verwendet wird. Dieses Material besteht z.B. aus Polylactid, in das eine Matrix aus Polyurethan angelegt ist. Dieses biodegradierbare Material kann zur Herstellung von porösen Membranen verwendet werden, mit denen große Wunden bedeckt werden können. Die Poren des Materials werden je nach Anwendung zwischen 10 und etwa 100 Mikrometern (µ) eingestellt. Dieses Material wird allerdings nicht dazu verwendet, Biomaterial zu beschichten.

Aus der EP-A1-0 578 998 ist es bekannt, implantierbare Endoprothesen mit einer Umkleidung aus einem biologisch abbaubaren Material zu versehen, wobei das Beschichtungsmaterial plastisch verformbar ist, damit bei der Veränderung des Querschnittes der Prothese das Beschichtungsmaterial nicht reißt, sondern den Formveränderungen folgt. Hiermit soll im wesentlichen eine Stabilisierung der Prothese im implantierten Zustand möglich sein, wobei auch bei einer maximalen Aufweitung der Prothese eine durchgängige Beschichtung durch die Umkleidung gewährleistet wird. Durch das biologisch abbaubare Beschichtungsmaterial können in dem Beschichtungsmaterial eingebettete Medikamente durch Freisetzung beim Abbau des Beschichtungsmateriales lokal in Kontakt mit Körperflüssigkeiten kommen.

Aus der EP-A1-0 562 612 ist ein biologisch abbaubares Beschichtungsmaterial beschrieben, das wiederum dazu dient, Prothesen flüssigkeitsdicht zu umhüllen. Auch in dieses Beschichtungsmaterial können Medikamente eingearbeitet sein, die dann beim Abbau der Beschichtung freigesetzt werden.

Der Erfindung liegt die Aufgabe zugrunde, die Beschichtung für die genannten Biomaterialien so zu modifizieren, daß eine Anlagerung von Blutgerinnseln aufgrund von plasmatischer oder zellulärer Blutgerinnung in hohem Maße vermieden wird; außerdem soll es möglich sein, auch weitere, z.B. blutgerinnungs- oder entzündungshemmende Wirkstoffe in die Beschichtung einbauen zu können, die dann auch ausreichend an das umgebende Gewebe bzw. den Blutstrom abgegeben werden.

Diese Aufgabe ist gemäß der Erfindung durch die im kennzeichnenden Teil des Patentanspruches 1 angegebenen Merkmale gelöst.

Demgemäß besteht der überraschend einfache Gedanke der Erfindung darin, Biomaterial mit einer dünnen, lackartigen Beschichtung zu benetzen, die im Körper, z.B. im Gewebe bzw. im Blutstrom permanent degradiert wird. Die Degradation kann hydrolytisch und/oder enzymatisch oder durch sonstige Zerfallsprozesse erfolgen. Entscheidend hierbei ist, daß die Schichtdicke des degradier- bzw. resorbierbaren Beschichtungsmaterials kleiner als 100 Mikrometer, insbesondere kleiner als 50 Mikrometer oder kleiner als 10 Mikrometer sind. Bevorzugt liegen die Schichtdicken unter 10 Mikrometer. Durch derartig dünne lackartige Beschichtungen wird die Oberfläche des Biomaterials quasi nur benetzt, wohingegen die Primärstruktur des Gebildes aus dem Biomaterial im wesentlichen nicht verändert wird. Wenn demnach z.B. ein poröses Material, etwa die erwähnten Stents, derartig beschichtet werden, so wird nur die Primärstruktur, demnach die Stränge des Maschengewebes des Stents beschichtet, d.h. daß allgemein bei porösen Materialien die Poren nicht verschlossen werden, sondern nur die Primärstruktur ummantelt wird. Die Porosität bleibt somit erhalten. Mit einer solchen Beschichtung wird demnach eine poröse Struktur nicht abgedichtet. Bei Kathetern oder anderen Biomaterialien mit geschlossener Oberfläche wird quasi nur die gesamte Oberfläche benetzt, so daß eine nur dünne Barriere von etwa 10 Mikrometern und kleiner zwischen dem Biomaterial und dem Blut bzw. dem Körpergewebe geschaffen wird.

Für eine derartige lackartige Beschichtung mit den erwähnten kleinen Schichtdicken können nur resorbierbare Materialien, z.B. Kunststoffe verwendet werden, die in schnellflüchtigen Lösungsmitteln gelöst, dispergiert oder emulgiert sind, so daß sich beim Beschichten nur eine Ummantelung der Primärstruktur des Biomaterials ergibt, ohne daß etwa die Beschichtung abblättert.

Vorliegende Untersuchungsergebnisse ex vivo sowie in vivo haben gezeigt, daß unbeschichtete Biomaterialien, wie bereits aufgezählt, nach einem Blutkontakt von nur wenigen Minuten mit einem dicken Blutgerinnsel überzogen sind. Bereits die alleinige Oberflächenbeschichtung dieser Materialien mit im Körper degradierbaren Beschichtungsmaterialien verhindert durch die permanenten Abbauvorgänge im gewissen Umfang bereits die Anhaftung von Blutgerinnseln. Der permanente Abbau oder Zerfall des Beschichtungsmaterials erlaubt nur eine zeitlich begrenzte Haftung der entsprechenden Blutbestandteile, wie Albumin, adhäsiver Proteine und Thrombozyten etc.

Derartige für die Beschichtung geeignete Materialien sind an sich bekannt, einige werden bereits im Rahmen der verzögerten Freisetzung von Arzneistoffen im Körper eingesetzt. Zur Erzielung gleichmäßiger Wirkspiegel enteral verabreichter Wirkstoffe haben sich im Magen-Darmtrakt schwerlösliche Überzüge von Kapseln, Dragees, Pulvern oder anderer galenischer Formen mittels Gelatine, Zellulose und (Meth)Acrylsäure und deren Derivaten neben anderen Hilfsmitteln bewährt. Mit der Entwicklung von im Körper abbaubaren synthetischen Polymeren, die als Arzneistoffträger einsetzbar sind, ergeben sich neue Wege der kontrollierten Freisetzung. So kann man heute auf einen umfangreichen Katalog verschiedenartiger Materialien zurückgreifen, die sich in der Struktur, den physikochemischen Eigenschaften und der Degradation im Körper unterscheiden. Bekannt ist auch die Einarbeitung von Wirkstoffen in diese Materialien, die dann als Trägermaterial dienen und die Ausbildung von Pulvern, Mikrokugeln sowie anderen Arzneistofformen. Nach der meist einmaligen subkutanen intramuskulären Injektion oder der Implantation z.B. unter die Haut werden über längere Zeit somit gleichmäßige Wirkspiegel der eingearbeiteten Arzneistoffe beobachtet.

Derartige Materialien können als Beschichtungsmaterial für die hier infrage stehenden Biomaterialien verwendet werden. Bevorzugt werden als biodegradierbares Beschichtungsmaterial Gele, Polyglykole oder Polylaktide verwendet. Beispielhaft seien noch genannt: biodegradierbare synthetische Polymere, wie Polyglykole und Polylaktide sowie entsprechende Mischpolymerisate oder Mischungen, Polyhydroxybutyrate und Polyhydroxyvaleriate sowie entsprechende Mischpolymerisate und Polydioxanon, ferner Stärke, Gelatine, Zellulose, Polyglykolsäure, Acrylsäure und Methacrylsäure sowie deren Derivate.

Das Beschichtungsmaterial muß fest auf der Oberfläche des Biomaterials, z.B. im Sinne einer nur dünnen Lackschicht, haften, die die Struktur und Funktion des Biomaterials nicht verändert. Die Beschichtungsmaterialien werden bevorzugt durch ein Tauchverfahren auf das Biomaterial aufgebracht und anschließend getrocknet.

Das Beschichtungsmaterial wird bevorzugt als Trägermaterial für einzuarbeitende Arzneistoffe verwendet, so daß diese Arzneistoffe synchron mit der Degradation des Trägermaterials abgegeben werden.

Bevorzugt werden in das als Trägermaterial dienende Beschichtungsmaterial Wirkstoffe zur Blutgerinnungshemmung, vorzugsweise zwei Wirkstoffe eingebaut, die die plasmatische und die zelluläre Blutgerinnung hemmen oder verhindern. Nach der Einarbeitung derartiger blutgerinnungshemmender Arzneistoffe, so z.B. Hirudin, konnten auf dem Biomaterial nur noch sehr vereinzelt Fibrinfäden nachgewiesen werden, die anhand der Rasterelektronenmikroskopie detektiert wurden. Vergleichbare Befunde wurden mit natürlichen bzw. synthetischen Prostaglandinderivaten erhoben. Durch die Kombination von Hirudin mit natürlichen bzw. synthetischen Prostaglandinderivaten konnten auch nach längerer Verweildauer des Biomaterials im Körper mit der Rasterelektronenmikroskopie weder Proteinablagerungen oder Fibringerinnsel noch aggregierte Thrombozyten nachgewiesen werden. Untersuchungen von Markern der aktivierten Gerinnung belegten diese Aussagen. Erhöhte Spiegel der Thrombin-Antithrombin-III-Komplexe (TAT-Komplexe) sowie der Prothrombinfragmente F₁₋₂ waren nur nach Blutkontakt mit den unbeschichteten Materialien, jedoch nicht mehr nach Beschichtung gemäß der Erfindung nachweisbar. Die unmittelbar mit den beschichteten Biomaterialien in Berührung gekommenen Gefäßwandstrukturen geben anhand der feingewebtichen und rasterelektronenmikroskopischen Untersuchungen keine Hinweise auf Veränderungen der Zellstruktur der Gefäßinnenwände. Eine Hyperplasie konnte nicht nachgewiesen werden. Auch Materialien, die unter nicht aseptischen Bedingungen hergestellt wurden, ergaben nach der Implantation in die Arteria femoralis keine Anzeichen einer Bakterienbesiedelung.

Weitere Verbesserungen konnten durch den Einbau von antiadhäsiven Peptiden entweder allein oder in Kombination mit den bereits genannten blutgerinnungshemmenden Substanzen bzw. mit Thrombozytenaggregationshemmern oder mit Thrombozytenrezeptorantagonisten erreicht werden. Hierdurch wird die Anhaftung der Gerinnungsproteine, der Fibrinfäden des Blutgerinnsels sowie der Thrombozyten unterbunden. Es fehlen dann die für die Adhäsion und Aggregation der Thrombozyten notwendigen adhäsiven Proteine, die als Brückenproteine fungieren, wobei die freigesetzten Thrombozytenaggregationshemmer und/oder die Thrombozytenrezeptorantagonisten die Adhäsion und Aggregation verhindern. Die hohen, lokal wirkenden Fibrinolytikakonzentration lysieren gebildete Blutgerinnsel und führen weiter zu einer kontinuierlichen Selbstreinigung der Oberflächen.

Eine mögliche überschießende Gewebeneubildung wird durch gleichzeitig vorhandene antiproliferative Wirkstoffe verhindert. Hier sind Corticoide zu nennen, um eine entzündungsbedingte Freisetzung von Gerinnungsfaktoren sowie von entsprechenden Mediatoren zu verhindern.

Auch der gleichzeitige Einsatz von gefäßrelaxierenden Substanzen ist angezeigt. Es ist bekannt, daß bei einer Schädigung des Endothels körpereigene Substanzen, die im normalen Gewebe eine Gefäßerweiterung bewirken, dann eine gefäßzusammenziehende Wirkung bewerkstelligen. Die lokale Gefäßtonisierung läßt sich durch die Einarbeitung von Stickstoffmonoxid (NO) bzw. von Pharmaka, die Stickstoffmonoxid freisetzen, wie organische Nitrate oder Molsidomin, in das Trägermaterial erreichen.

Somit ist es möglich, in dem Klinikbetrieb bereits bewährte Biomaterialien mit blutverträglichen, antiproliferativen und antibiotischen Oberflächen zu überziehen, und zwar speziell orientiert an den Bedürfnissen für den jeweiligen Patienten. Durch die kontinuierliche Selbstreinigung der Oberflächen bei gleichzeitig hoher Dosierung lokal wirkender Arzneistoffe werden die Haftung von Plasmaproteinen, Ausbildung von Gerinnungsfaktoren sowie Gerinnungsvorgänge und auch die Aggregation von Thrombozyten verhindert.

Durch den Einbau von mehreren Wirkstoffen gegen Blutgerinnung und/oder Entzündung werden synergistische Effekte erreicht, die auf die jeweiligen Bedürfnisse exakt abgestimmt werden können.

Die Beschichtung von Biomaterial gemäß der Erfindung kann für Instrumente und Geräte oder dergleichen eingesetzt werden, die nur kurz im Körpergewebe bzw. im Blutstrom eingebracht sind, wie Katheter, Nahtmaterial etc., aber auch für Materialien, die lange Zeit bzw. lebenslang im Körper verbleiben, so z.B. die oben erwähnten Stützkörper bzw. Stents. Auch wenn nach einer gewissen Zeit, die mit Hilfe des gewählten Beschichtungsmaterials und der Beschichtungsdicke etc. eingestellt werden kann, nach einiger Zeit, z.B. einigen Wochen, das Beschichtungsmaterial vollständig abgebaut sein sollte, ergeben sich in aller Regel keine unverträglichen Nebenwirkungen mehr, da dann das im Körper eingebrachte Biomaterial mit einer körpereigenen Gewebe- bzw. Zellschicht versehen ist. Ab diesem Zeitpunkt ist auch eine weitere Blutgerinnung durch Anlagerung oder eine weitere Entzündung nicht mehr zu befürchten.

Das Beschichtungsmaterial kann auf das Biomaterial in einer oder in mehreren Schichten aufgebracht werden, wobei dann, sofern Arzneistoffe eingearbeitet sind, diese Arzneistoffe nur in eine der Schichten, in verschiedenen Schichten oder in allen Schichten enthalten sein können. Bei mehrlagigen Beschichtungen können die einzelnen Schichten aus unterschiedlichen Beschichtungsmaterialien bzw. Trägermaterialien bestehen, die gegebenenfalls auch unterschiedliche Degradationsgeschwindigkeiten aufweisen. Die Schicht mit der längsten Verweildauer im Körper wird dann in der Regel als unterste Schicht angeordnet. Ein Arzneistoff kann dann z.B. in dieser Schicht eingearbeitet sein. Die Arzneistoffe können auch als Pulver oder Kristalle in dem Beschichtungsmaterial suspendiert oder in der lackartigen Oberflächenbeschichtung homogen verteilt sein.

Im folgenden werden Beispiele für eine Oberflächenbeschichtung mit blutverträglichen, antiproliferativen, entzündungshemmenden sowie antibiotischen Eigenschaften erwähnt, wobei die Arzneiwirkstoffe im Beschichtungs- bzw. Trägermaterial suspendiert und/oder homogen verteilt sind.

Zunächst wird eine **Grundlösung** für ein Beschichtungsmaterial hergestellt: Hierzu werden 480 mg eines Arzneistoffträgers, wie Poly-D,L-Laktid (käuflich als R203 der Firma Boehringer, Ingelheim) in 3 ml Chloroform unter aseptischen Bedingungen gelöst.

Für Grundlösungen eignen sich alle oben genannten biodegradierbaren Beschichtungsmaterialien in Lösung, Dispersion oder Emulsion, die nach Trocknung selbsthaftende, lackartige Überzüge mit den angegebenen dünnen Schichtdicken ergeben.

In diese Grundlösung für das Beschichtungs- bzw. Trägermaterial können dann noch sterile Wirkstoffe, wie nachfolgend beschrieben, zugegeben werden, um jeweils ein spezifisches Beschichtungsmaterial zu erhalten.

Für eine **Hirudinbeschichtung** werden 24 mg fein verteiltes Hirudinpulver unter aseptischen Bedingungen in der Grundlösung dispergiert und bis zur Beschichtung bei -10°C gelagert. Anstelle eines Hirudinpulvers kann eine entsprechende arzneistoffhaltige Lösung dispergiert werden; möglich sind auch andere blutgerinnungshemmende analoge Substanzen, wie Hirolog. Es ist auch möglich, das Hirudinpulver oder entsprechende andere Arzneistoffe in einer bereits arzneistoffhaltigen Grundlösung zu dispergieren.

Für eine Beschichtung mit einem synthetischen Prostaglandinderivat **(Iloprostbeschichtung)** werden 0,48 mg Iloprost unter aseptischen Bedingungen in der Grundlösung oder einer entsprechenden arzneistoffhaltigen Lösung gelöst und bis zur Beschichtung bei -10°C gelagert.

Für eine **Dexamethasonbeschichtung** werden 4,8 mg fein verteiltes Dexamethasonpulver unter aseptischen Bedingungen in der Grundlösung oder einer entsprechenden arzneistoffhaltigen Lösung dispergiert und bis zur Beschichtung bei -10°C gelagert.

Für eine **Gentamicinbeschichtung** werden 4,8 mg fein verteiltes Gentamicinpulver unter aseptischen Bedingungen in der Grundlösung oder einer entsprechenden arzneistoffhaltigen Lösung dispergiert und bis zur Beschichtung bei -10°C gelagert.

Für eine **Heparinbeschichtung** werden 24 mg fein verteiltes Heparinpulver unter aseptischen Bedingungen in der Grundlösung oder einer entsprechenden arzneistoffhaltigen Lösung dispergiert und bis zur Beschichtung bei -10°C gelagert.

Für eine **Urokinasebeschichtung** werden 200 000 Einheiten fein verteiltes Urokinasepulver unter aseptischen Bedingungen in der Grundlösung oder einer entsprechenden arzneistoffhaltigen Lösung dispergiert und bis zur Beschichtung bei -10°C gelagert.

Zur **Beschichtung** wird das jeweilige Biomaterial unter aseptischen Bedingungen bei etwa Zimmertemperatur in die jeweilige klebrige Lösung getaucht, wonach das beschichtete Material dann bei etwa 40°C getrocknet wird. Es zeigte sich, daß mit den angegebenen Materialien tatsächlich nur die Primärstruktur des Biomaterials mit lackartigen Schichten mit Dicken kleiner als 100 Mikrometern benetzt wird, d.h. daß die äußerliche Struktur und Form im wesentlichen erhalten bleibt.

Mit derartigen Lösungen wurden handelsübliche Gefäßstützen, die sogenannten Stents, Gefäßprothesen, Karbonfasergeflechte, Schrittmacherelektroden, Defibrillatorelektroden, diagnostische und therapeutische Plastikkatheter für Angiologie und Kardiologie ohne und mit Ballon, Schläuche und Beutel wie in der Transfusionsmedizin gebräuchlich, Schlauchsysteme von Herz-Lungenmaschinen, entsprechende Oxigenatoren, Plastikmandrins von Braunülen sowie Arterienkanülen oder Nahtmaterial für Gefäßanastomosen beschichtet. Die Beschichtung haftete auf allen diesen Materialien einwandfrei und baut sich im Blutstrom oder in dem Gewebe in gewünschter Weise permanent ab. Dieser Abbau wurde in Tests ex vivo und in vivo überprüft.

Das Testsystem ex vivo, als Modell eines extrakorporalen Kreislaufes, bestand aus einer Braunüle zur Blutabnahme aus der menschlichen Cubitalvene, an die ein paralleles Schlauchsystem angeschlossen war, das aus sechs ca. fünf Zentimeter langen Schlauchstücken bestand, die aus Zuleitungen von Blutkonserven hergestellt waren. In diese Schläuche wurden entsprechend beschichtete und unbeschichtete Biomaterialien eingebracht. Durch Blutabnahme am Ende der jeweiligen Schläuche wurde sichergestellt, daß die Biomaterialien mit Blut in Kontakt kamen. An jeden der Schläuche konnte zeitabhängig nach dem Blutkontakt mit einer Spritze Blut abgenommen werden. In den so gewonnenen Blutproben hat sich die Untersuchung der TAT-Komplexe sowie der Prothrombinfragmente F₁₋₂ als biochemische Marker für die aktivierte Gerinnung bewährt. Nach dem Blutkontakt wurden die Materialien herausgenommen und makroskopisch sowie mikroskopisch auf die Anwesenheit gebildeter Blutgerinnsel untersucht. Eine weitere Charakterisierung etwaiger Gerinnsel erfolgte mit Hilfe der Rasterelektronenmikroskopie, und zwar hinsichtlich Fibrinablagerungen und aggregierten Thrombozyten.

Für einen Test in vivo wurden z.B. Plastikmandrins der Braunülen oder der Arterienkatheter beschichtet und in die Femoralarterien von nicht antikoagulierten Tieren eingebracht. Als Kontrolle dienten unbeschichtete bzw. nur mit dem im Körper abbaubaren Beschichtungsmaterial beschichtete Mandrins. Nach Beendigung der Versuche wurden die Materialien herausgenommen und makroskopisch sowie mikroskopisch und rasterelektronenmikroskopisch auf Blutgerinnsel untersucht. Die mit den Mandrins in Berührung gekommenen Venenwände wurden mit Hilfe der Rasterelektronenmikroskopie und verschiedenen histologischen Methoden hinsichtlich Fibrinablagerungen, aggregierter Thrombozyten sowie Gewebereaktionen der Gefäßwände beurteilt.

## Patentansprüche

1. Beschichtung für in den Blutstrom oder in das Gewebe des menschlichen Körpers einbringbares Biomaterial, wie Infusions-, Herz- oder Ballonkatheter, Elektroden für Herzschrittmacher und Defibrillatoren, Nahtmaterial, Stützkonstruktionen für Gefäße (Stents) oder dergleichen, wobei diese Beschichtung aus einem blut- und gewebeverträglichen und in dem Körper permanent degradierbaren Beschichtungsmaterial besteht, **dadurch gekennzeichnet, dass** das Beschichtungsmaterial so gewählt ist, dass durch die Beschichtung die Blutgerinnung an dem Biomaterial durch Anhaften von plasmatischen oder zellulären Bestandteilen verhindert wird, dass das Beschichtungsmaterial ein auf dem Biomaterial selbsthaftendes Beschichtungsmaterial ist, und dass das Beschichtungsmaterial auf dem Biomaterial eine lackartige Haftschicht mit Schichtdicken kleiner als 100 Mikrometern (< 100 µm) bildet .

2. Beschichtung nach Anspruch 1, **dadurch gekennzeichnet, dass** das Beschichtungsmaterial eine Schichtdicke von kleiner als 50 µm aufweist.

3. Beschichtung nach Anspruch 1 oder 2, **dadurch gekennzeichnet, dass** das Beschichtungsmaterial eine Schichtdicke von kleiner als 10 µm aufweist.

4. Beschichtung nach einem der vorhergehenden Ansprüche, **dadurch gekennzeichnet, dass** das Beschichtungsmaterial hydrolytisch und/oder enzymatisch und/oder durch sonstige Zerfallsprozesse permanent degradiert wird.

5. Beschichtung nach einem der vorhergehenden Ansprüche, **dadurch gekennzeichnet, dass** das Beschichtungsmaterial ein biodegradierbares synthetisches Polymer ist.

6. Beschichtung nach einem der vorhergehenden Ansprüche, **dadurch gekennzeichnet, dass** das Beschichtungsmaterial zunächst als Lösung, Dispersion oder Emulsion in einem schnellflüchtigen Lösungsmittel mit hoher Oberflächenspannung vorliegt und nach Aufbringen auf das Biometrial und anschließender Trocknung eine trockene dünne Haftschicht bildet.

7. Beschichtung nach einem der vorhergehenden Ansprüche, **dadurch gekennzeichnet, dass** in das Beschichtungsmaterial Arzneistoffe eingearbeitet sind.

8. Beschichtung nach Anspruch 7, **dadurch gekennzeichnet, dass** in das Beschichtungsmaterial zusätzliche, die Blutgerinnung hemmende Arzneistoffe eingearbeitet sind.

9. Beschichtung nach Anspruch 8, **dadurch gekennzeichnet, dass** die Arzneistoffe die plasmatische und zelluläre Blutgerinnung hemmende Stoffe sind.

10. Beschichtung nach Anspruch 7, **dadurch gekennzeichnet, dass** in das Beschichtungsmaterial entzündungshemmende Arzneistoffe eingearbeitet sind.

11. Beschichtung nach Anspruch 10, **dadurch gekennzeichnet, dass** die entzündungshemmenden Arzneistoffe Cortikoide sind.

12. Beschichtung nach Anspruch 7, **dadurch gekennzeichnet, dass** in das Beschichtungsmaterial Antibiotika eingearbeitet sind.

13. Beschichtung nach Anspruch 7, **dadurch gekennzeichnet, dass** in das Beschichtungsmaterial gefäßrelaxierende Arzneistoffe eingearbeitet sind.

14. Beschichtung nach einem der vorhergehenden Ansprüche, mehrere Schichten aus ggf. unterschiedlichen Beschichtungsmaterialien vorgesehen sind.

15. Beschichtung nach Anspruch 14, **dadurch gekennzeichnet, dass** die Schicht mit der längsten Verweildauer im Körper die unterste Schicht bildet.

## Claims

1. Coating for bio-material which can be introduced into the bloodstream or into the tissue of the human body, such as infusion, heart or balloon catheters, electrodes for heart pacemakers and de-fibrillators, suture material, stents or the like, with this coating consisting of a coating material well tolerated by blood and tissue and permanently degradable in the body, **characterized in that** the coating material is selected so that blood clotting on the bio-material due to constituents of plasma or cells sticking to it being prevented by this coating, that the coating material on the bio-material is a pressure-sensitive coating material, and that the coating material forms a paint-like adhesive layer on the bio-material, with layer thicknesses of less than 100 micrometres (< 100 µm).

2. Coating material as Claim 1, characterized that the coating material has a layer thickness of under 50 µm.

3. Coating material as Claim 1 or 2, **characterized in that** the layer thickness of the coating material is less than 10 µm.

4. Coating material as one of the previous claims, **characterized in that** the coating material is permanently degraded hydrolytically and / or enzymatically and / or through other disintegration processes.

5. Coating as one of previous claims, **characterized in that** the coating material is a bio-degradable synthetic polymer.

6. Coating as one of previous claims, **characterized in that** the coating material is initially present as a solution, dispersion or emulsion in a rapidly volatising solvent with a high surface tension, and forms a dry, thin adhesive layer following application to the bio-material and subsequent drying.

7. Coating as one of previous claims, **characterized in that** medicaments are incorporated into the coating material.

8. Coating as one of previous claims, **characterized in that** additional medicaments inhibiting blood clotting are incorporated into the coating material.

9. Coating as Claim 8, **characterized in that** the medicaments are components for inhibiting plasma and cellular blood clotting.

10. Coating as Claim 7, **characterized in that** medicaments inhibiting inflammation are incorporated into the coating material.

11. Coating as Claim 10, **characterized in that** the inflammation-inhibiting medicaments are corticoids.

12. Coating as Claim 7, **characterized in that** anti-biotics are incorporated into the coating material.

13. Coating as Claim 7, **characterized in that** vascular relaxing medicaments are incorporated into the coating material.

14. Coating as one of previous claims, **characterized in that** several layers are provided for, which may be of different coating materials.

15. Coating as Claim 14, **characterized in that** the layer which lasts longest in the body forms the bottom layer.

## Revendications

1. Revêtement pour matériel biologique à insérer dans le sang ou dans les tissus du corps humain, tel que des cathéters d'infusion, cardiaques ou à ballon, des électrodes pour des pacemakers et défibrillateurs, du matériel de suture, des structures de support des vaisseaux (écarteurs) ou similaires, ce revêtement étant constitué d'un matériau de revêtement compatible avec le sang et les tissus, et à décomposition continue par le corps, **caractérisé en ce que** ce matériau de revêtement est choisi de cette façon que par ce matériau de revêtement la coagulation du sang sur le matériel biologique par l'adhérence de composantes plasmatiques ou cellulaires est empêchée, que le matériau de revêtement adhère de lui-même au matériel biologique et que le matériau de revêtement constitue sur le matériel biologique une couche d'adhésion sous forme de laque d'épaisseur inférieure à 100 micromètres (< 100 µm).

2. Revêtement selon la revendication 1, **caractérisé en ce que** le matériau de revêtement présente une épaisseur inférieure à 50 µm.

3. Revêtement selon la revendication 1 ou 2, **caractérisé en ce que** l'épaisseur du matériau de revêtement est inférieure à 10 µm.

4. Revêtement selon l'une quelconque des précédentes revendication **caractérisé en ce que** le matériau de revêtement est décomposé continûment de manière électrolytique, enzymatique et/ou par d'autres processus de décomposition.

5. Revêtement selon l'une quelconque des revendications précédentes, **caractérisé en ce que** le matériau de revêtement est un polymère synthétique biodégradable.

6. Revêtement selon l'une quelconque des revendications précédentes, **caractérisé en ce que** le matériau de revêtement est d'abord prévu en tant que solution, dispersion ou émulsion dans un solvant volatile à tension superficielle élevée et constitue, après application sur le matériel biologique et un séchage subséquent, une fine couche d'adhésion sèche.

7. Revêtement selon l'une quelconque des revendications précédentes, **caractérisé en ce que** le matériau de revêtement comporte des médicamentes.

8. Revêtement selon la revendication 7, **caractérisé en ce que** le matériau de revêtement contient des additionels produits anticoagulants.

9. Revêtement selon la revendication 8, **caractérisé en ce que** le matériau de revêtement es un produit pour empêcher la coagulation plasmatique et cellulaire.

10. Revêtement selon la revendication 7, **caractérisé en ce que** le matériau de revêtement contient des produits anti-inflammatoires.

11. Revêtement selon la revendication 10, **caractérisé en ce que** les produits anti-inflammatoires contiennent des corticoïdes.

12. Revêtement selon la revendication 7, **caractérisé en ce que** le matériau de revêtement contient des antibiotiques.

13. Revêtement selon la revendication 7, **caractérisé en ce que** le matériau de revêtement comprend des produit de relaxation des vaisseaux.

14. Revêtement selon l'une quelconque des revendications précédentes, **caractérisé en ce qu'**il comprend plusieurs couches, le cas échéant de différents matériaux de revêtement.

15. Revêtement selon la revendication 14, caractérisé∼ en ce que la couche ayant la durée de vie la plus longue dans le corps constitue la couche inférieure.
